# EUROPEAN PATENT APPLICATION

(11) **EP 1 304 635 A1**
(43) Date of publication of application: **23.04.2003**
(21) Application number: 01947982.3
(22) Date of filing: 12.07.2001
(51) Int. Cl.: G06F 17/60, A61B 5/00

(54) **MEDICAL INFORMATION SYSTEM, PATIENT-USE TERMINAL DEVICE, MEDIUM**

(30) Priority: 14.07.2000 JP 2000214470
(71) Applicant: MATSUSHITA ELECTRIC INDUSTRIAL CO., LTD., Kadoma-shi, Osaka 571-8501 (JP)
(72) Inventor: IWANO, Kenji, Osaka 576-0021 (JP); MIYAZAKI, Jinsei, Osaka 578-0901 (JP)
(74) Representative: Grünecker, Kinkeldey, Stockmair & Schwanhäusser Anwaltssozietät
(86) International application number: JP0106035
(87) International publication number: WO02007025

(57) **Abstract**

A system simply capable of browsing data, such as vital information of patients, was not available.

A medical information system comprising clients 201, a server 203 and hosts 202 connected on a network, wherein the client 201 has sensors for measuring information serving as indicators of the health condition of a patient and means of transferring vital information from the sensors to the server 203, the server 203 has means of statistically storing and arranging the vital information transferred from the client 201, and the host 202 has means of browsing the vital information stored and arranged in the server 203.

## Description

### Technical Field

The present invention relates to a system capable of browsing data regarding diseases and medicines.

### Background Art

The so-called aging society, wherein the life span of people is extending as the medical technology progresses but the proportion of old people in population increases at the same time, has become a worldwide issue. In the aging society, the rates of chronic diseases, such as diabetes, heart diseases and rheumatism, are high inevitably.

In this kind of contemporary society, various information and data regarding diseases and medicines become important.

However, a system capable of browsing such data and information easily and simply was not available conventionally.

### Disclosure of Invention

In consideration of this kind of conventional problem, the present invention is intended to provide a system and the like capable of collecting and simply browsing the vital information and the like of patients.

The present invention is a medical information system comprising patient terminal devices, a central control device and data browsing terminal devices, wherein
said patient terminal devices, said central control device and said data browsing terminal devices are connected on a network,
said patient terminal device has means of transferring vital information relating to the health condition of a patient to said central control device via said network and means of receiving control information from said central control device,
said central control device has means of storing said vital information transferred from said patient terminal device and arranging said information to information having anonymity, and
said data browsing terminal device has means capable of browsing all or parts of said vital information stored and arranged in said central control device.

### Brief Description of Drawings

FIG. 1 is a flowchart for a medical information system in accordance with a first embodiment of the present invention;
FIG. 2 is a view showing a configuration of the medical information system in accordance with the first embodiment of the present invention;
FIG. 3 is a flowchart indicating a procedure for browsing vital information;
FIG. 4 is a flowchart indicating a procedure for setting various conditions;
FIG. 5 is a view showing an output example of statistical data of vital information (blood pressure) ;
FIG. 6 is a view showing an output example of statistical data regarding the intake amount of breakfast;
FIG. 7 is a flowchart indicating a procedure for browsing vital information and charging money;
FIG. 8 is a flowchart indicating a procedure for browsing vital information and charging money on the basis of advertisement;
FIG. 9 is a view showing a configuration of a medical information system in accordance with a second embodiment of the present invention;
FIG. 10 is a flowchart indicating a procedure for medicine order reception/placement;
FIG. 11 is a view showing a configuration of a medical information system in accordance with a third embodiment of the present invention;
FIG. 12 is a flowchart indicating a procedure for registering information on the effects and side effects of medicines;
FIG. 13 is a flowchart indicating a procedure for browsing the information on the effects and side effects of medicines;
FIG. 14 is a view showing output example 1 of the information on the effects and side effects of medicines;
FIG. 15 is a view showing output example 2 of the information on the effects and side effects of medicines;
FIG. 16 is a flowchart indicating a procedure for browsing the information on the effects and side effects of medicines and charging money;
FIG. 17 is a flowchart indicating a procedure for browsing the information on the effects and side effects of medicines and charging money on the basis of advertisement;
FIG. 18 is a view showing a configuration of a medical information system in accordance with a fourth embodiment of the present invention;
FIG. 19 is a view showing a configuration of a client;
FIG. 20 is a view showing a configuration of a host;
FIG. 21 is a view showing a configuration of a server;
FIG. 22 is a view showing a network configuration of the medical information system in accordance with the first embodiment of the present invention; and
FIG. 23 is a view showing a network configuration of a medical information system in accordance with a fifth embodiment of the present invention.

### Explanation of Reference Numerals

- 201: client
- 202: host
- 203: server
- 901: guest
- 1101: medicine server
- 2201: HOSPITAL LAN
- 2202: PSTN
- 2203: RAS server

### Best Mode for Carrying Out the Invention

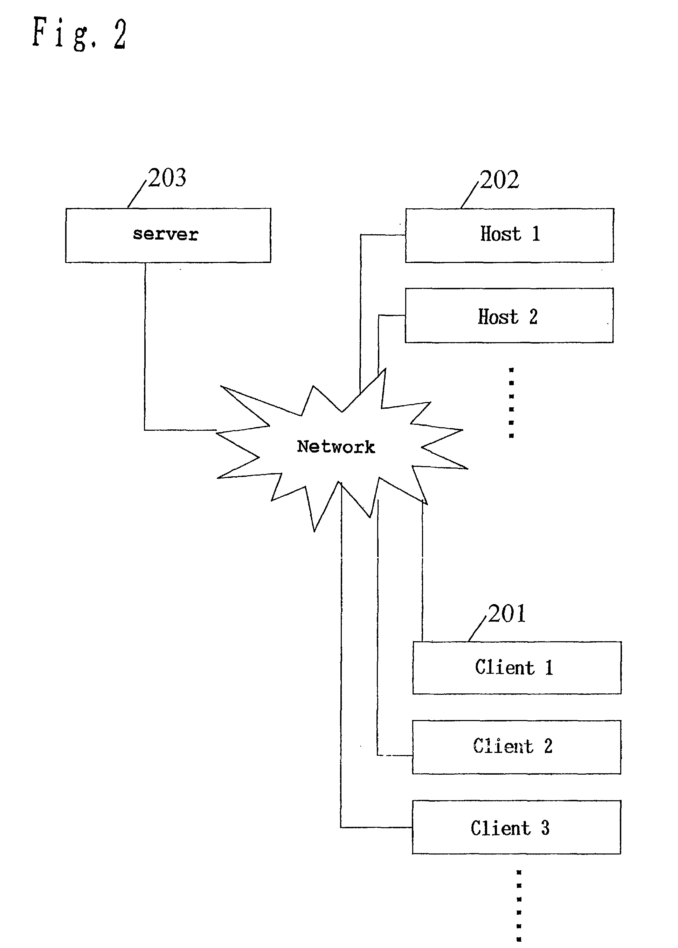

FIG. 2 is a view showing a configuration of a medical information system in accordance with a first preferred embodiment of the present invention.

In FIG. 2, the medical information system comprises patient terminal devices (hereafter simply referred to as clients) 201, a central control device (hereafter simply referred to as a server) 203 and administrator terminal devices (hereafter simply referred to as hosts) 202, and the above-mentioned clients 201, the above-mentioned server 203 and the above-mentioned hosts 202 are connected on a network. Herein, an administrator who uses the host 202 is a doctor or a nurse.

The above-mentioned client 201 has sensors having functions of measuring vital information serving as the indicators of the health condition of a patient, a means of transferring the above-mentioned vital information obtained by the above-mentioned sensors to the inside of the above-mentioned client and a means of transferring the above-mentioned vital information from the above-mentioned sensors to the above-mentioned server 203 via the network.

The above-mentioned server 203 has a means of storing the above-mentioned vital information transferred from the above-mentioned client 201 and arranging the information to information having anonymity, and the above-mentioned host 202 has a means of browsing the whole or part of the above-mentioned vital information stored or arranged in the above-mentioned server 203.

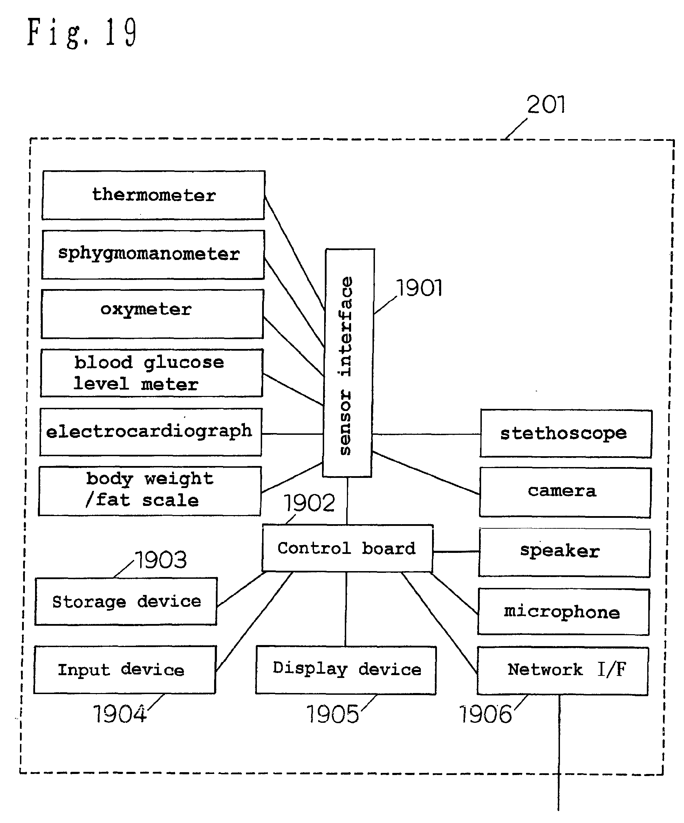

FIG. 19 is a view showing a configuration of the client 201. By using an input device 1904, a patient designates which sensor should be used; on the basis of this input, an control board 1902 extracts data regarding the method of using the sensor from a storage device 1903, and a display device 1905 displays the method of using the sensor.

The patient takes measurement by using the sensor in accordance with the instructions at the display device 1905 and obtains vital information. Herein, the above-mentioned sensors designate a thermometer, a sphygmomanometer, an oxymeter, a blood glucose level meter, an electrocardiograph, a body weight/fat scale, a stethoscope and a camera shown in FIG. 19, and include at least one of these.

The vital information obtained by the above-mentioned sensors is converted into a designated data sequence at a sensor interface 1901, the above-mentioned vital information is received by the control board 1902, and the vital information is stored in the storage device 1903 and displayed by the display device 1905 at the same time. However, not all sensors are required to be connected via the sensor interface 1901; in some cases, the vital information from the sensors can be received directly by the control board 1902.

The above-mentioned vital information stored in the storage device 1903 of the client 201 is transferred to the server 202 via a network I/F (an abbreviation for interface) 1906.

The input device 1904 includes at least one of a touch panel, a keyboard, a mouse and a voice input, the storage device 1903 includes at least one of a hard disk and a flash memory, and the network I/F 1906 includes at least one of an analog modem, an xDSL modem, a cable modem and an Ethernet adaptor.

Furthermore, in order that the client 201 realizes the function of a videoconference with the host 202, it is required to be provided with a camera, a microphone and a speaker.

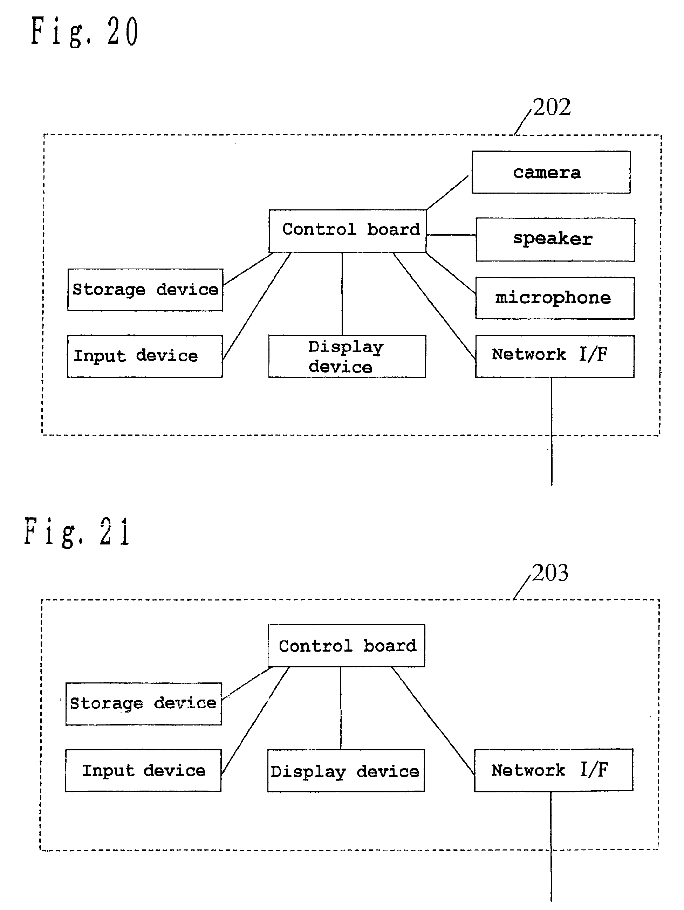

FIG. 20 is a view showing a configuration of the host 202. In order to realize a videoconference between the host 202 and the client 201, the host 202 is provided with a camera, a microphone and a speaker. In addition, it is also provided with a control board, a display device, a storage device and an input device.

FIG. 21 is a view showing a configuration of the server 203. It is provided with an interface for communication with the network, in addition to a control board, a display device, a storage device and an input device.

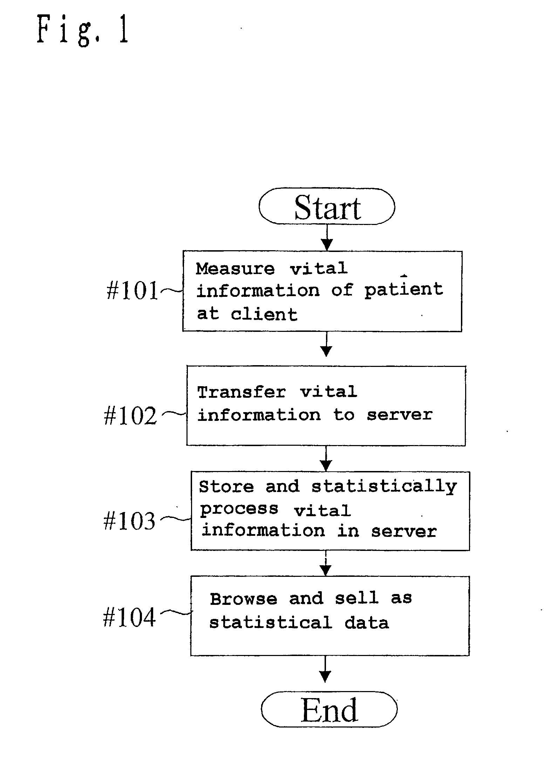

FIG. 1 is a flowchart for the medical information system having the configuration shown in FIG. 2.

At step #101, a patient measures vital information by using the client 201, and the above-mentioned vital information is stored in the inside of the client. At step #102, the above-mentioned vital information is transferred to the server 203.

At step #103, the transferred above-mentioned vital information is stored and arranged in the server 203. At step #104, the stored vital information is displayed only as statistical data. This is because, if the vital information is opened wholly, it is expected that human rights be violated significantly owing to an invasion of privacy. Hence, instead of personal level data, statistically processed data is browsed, whereby anonymity is secured, the issue of the invasion of privacy is avoided, and anyone is allowed to browse the tendency of the vital information.

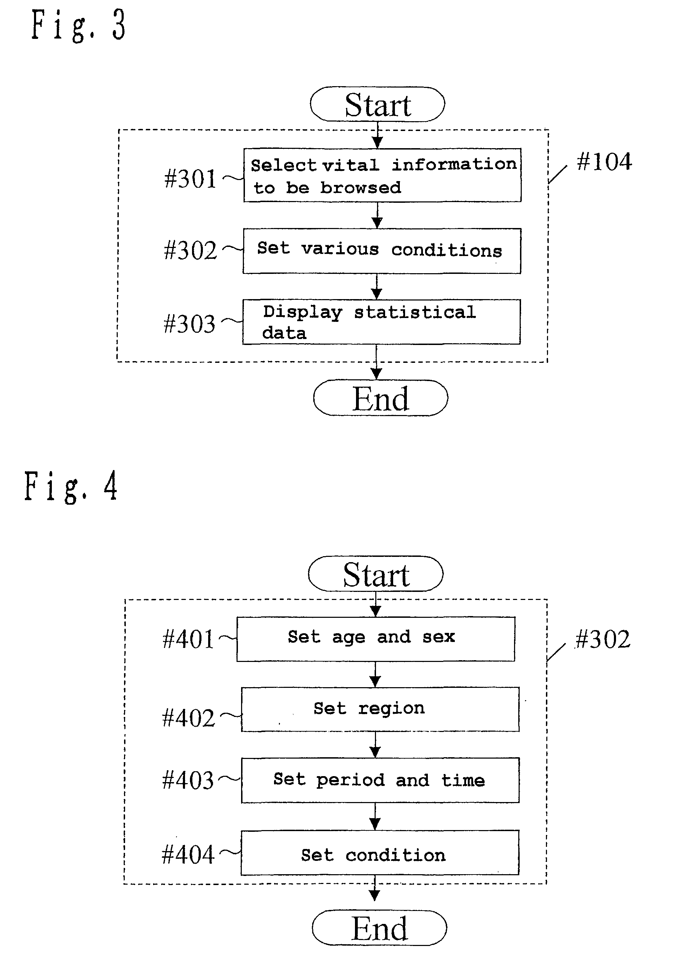

FIG. 3 is a detailed flowchart for step #104, and the process thereof starts after an administrator (a doctor or a nurse) wishing to browse the statistical data of the vital information makes a connection to the network by using the host 202 used as an example of the data browsing terminal device of the present invention and logs in to the server 203.

First, at step #301, the vital information to be browsed, for example, one of body temperature, blood pressure and blood glucose level, is selected. At step #302, the conditions of the vital information desired to be browsed are set.

FIG. 4 is a detailed flowchart for step #302. The age, sex, region, location, period (schedule), measurement time and condition of a patient are set as conditions. At step #303, the statistical data depending on the input conditions is output.

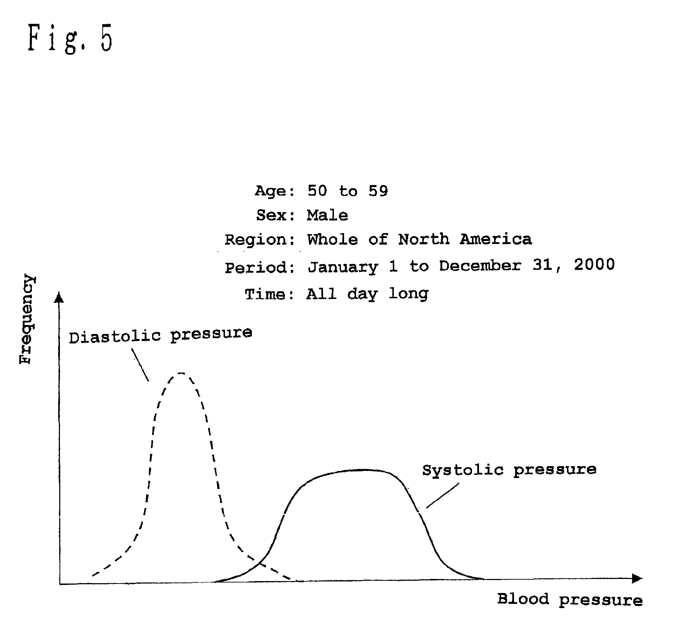

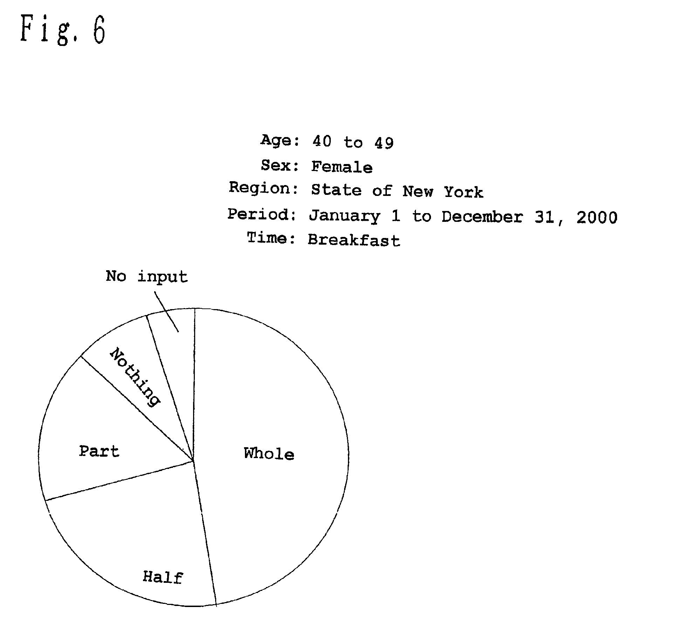

FIG. 5 shows an output example of the statistical data in the case when blood pressure is selected as the vital information. FIG. 6 shows an output example of the statistical data regarding the amount of breakfast taken by the patients. This data regarding the amount of breakfast is included in the vital information of the present information, and is supposed to be self-stated by people.

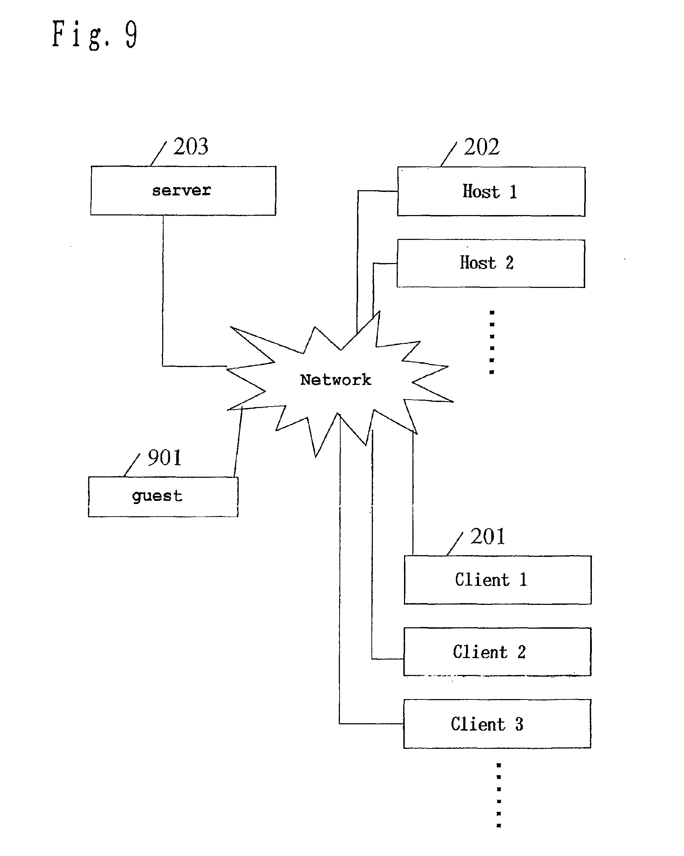

FIG. 9 is a view showing a configuration of a medical information system in accordance with a second preferred embodiment of the present invention.

This differs from FIG. 2 in that, in addition to the hosts 202 for doctors, a terminal device 901 (hereafter simply referred to as a guest) is connected to the network of the medical information system as an example of the data browsing terminal device of the present invention. In the above-mentioned explanation, an administrator (a doctor or a nurse) browses the statistical data; however, a third party (hereafter simply referred to as a user), not a patient or an administrator, that is, for example, a pharmaceutical researcher or the like, desires to brows the statistical data in some cases. In such a case, it is necessary to restrict the browsing of the statistical data or to charge money.

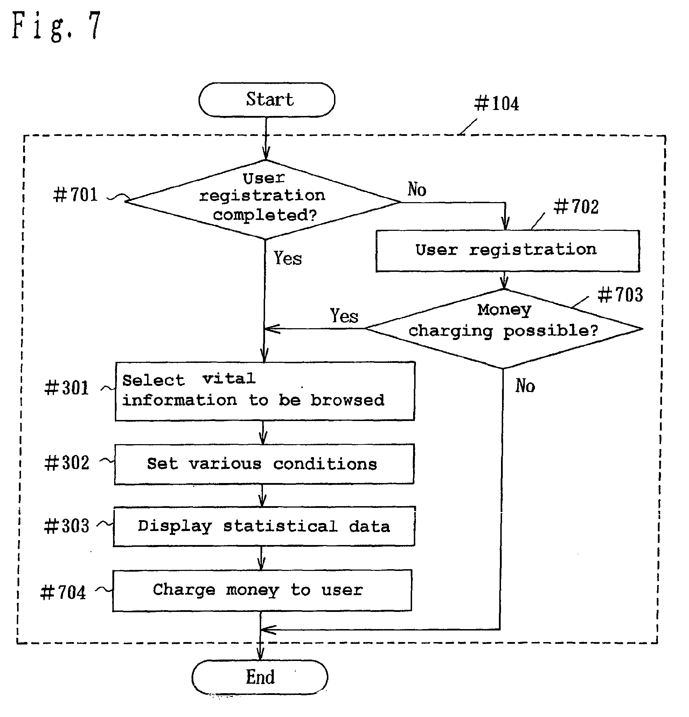

FIG. 7 is a detailed flowchart for step #104. In the flowchart shown in FIG. 3, data browsing is available at no charge; however, the flowchart shown in FIG. 7 differs in that a money charging process is added thereto.

At step #701, a confirmation is made as to whether a user wishing to browse the statistical data of vital information has made registration or not. If registration has been made, the sequence advances to step #301; if no registration has been made, the sequence advances to step #702. At step #702, user registration is made, and the money charging information for the user is collected.

The money charging information represents a credit card number, the personal identification number for a bank account, electronic money and the like.

At step #703, a judgment is made as to whether money charging is possible or not according to the money charging information obtained by the user registration; if money charging is possible, the sequence advances to step #301; if money charging is impossible, the processing ends.

The explanations for steps #301, #302 and #303 are omitted since they are similar to those given above.

After the statistical data is displayed, money is charged to the user at step #704. The money charging is performed depending on the amount of the statistical data having been browsed.

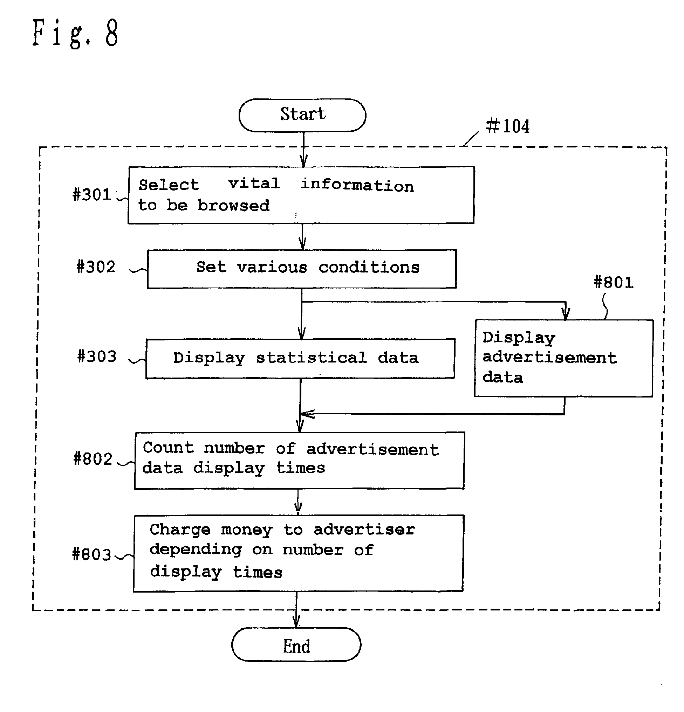

FIG. 8 is a detailed flowchart of another example for step #104. In the flowchart shown in FIG. 7, money is charged to a user; however in FIG. 8, money is charged to an advertiser.

This means that a contract is made with the advertiser in advance and that video/audio information desired by the advertiser is delivered to advertise his enterprise at the time when a user browses the statistical data.

The explanations for steps #301 and #302 are omitted since they are similar to those given above. At step #303, the statistical data is displayed, and advertisement data is also displayed. At step #802, the number of times the advertisement data is displayed is counted in a constant period (for example, one month). At step #803, money is charged to the advertiser depending on the number of display times.

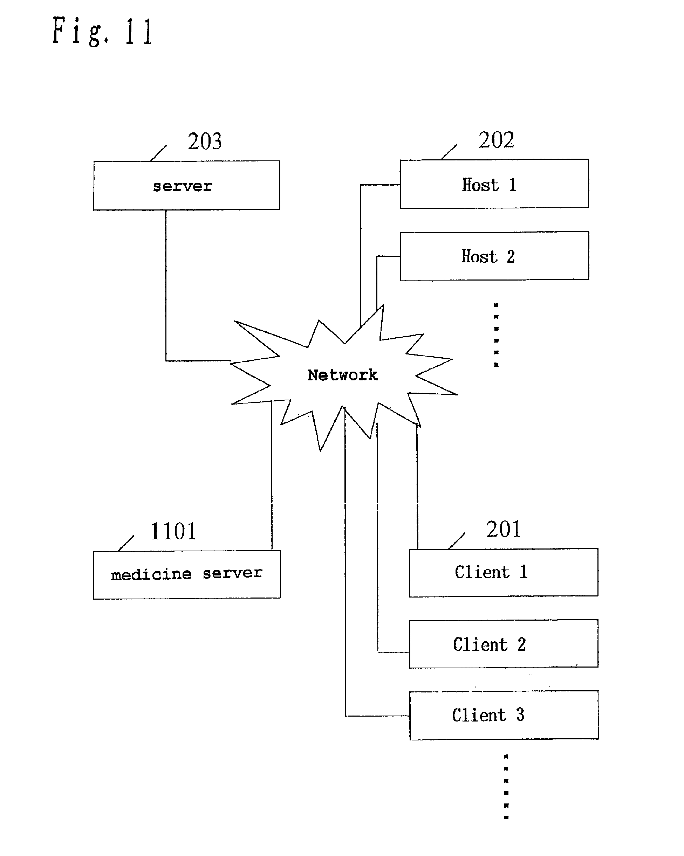

FIG. 11 is a view showing a configuration of a medical information system in accordance with a third preferred embodiment of the present invention. This differs from FIG. 2 in that a medicine control terminal device 1101 (hereafter simply referred to as a medicine server) is connected. This medicine server 1101 is installed at a pharmacy, a pharmaceutical company, etc. The device holds a database regarding medicines and can receive and place orders for medicines. This medicine server 1101 is installed at a pharmacy, for example.

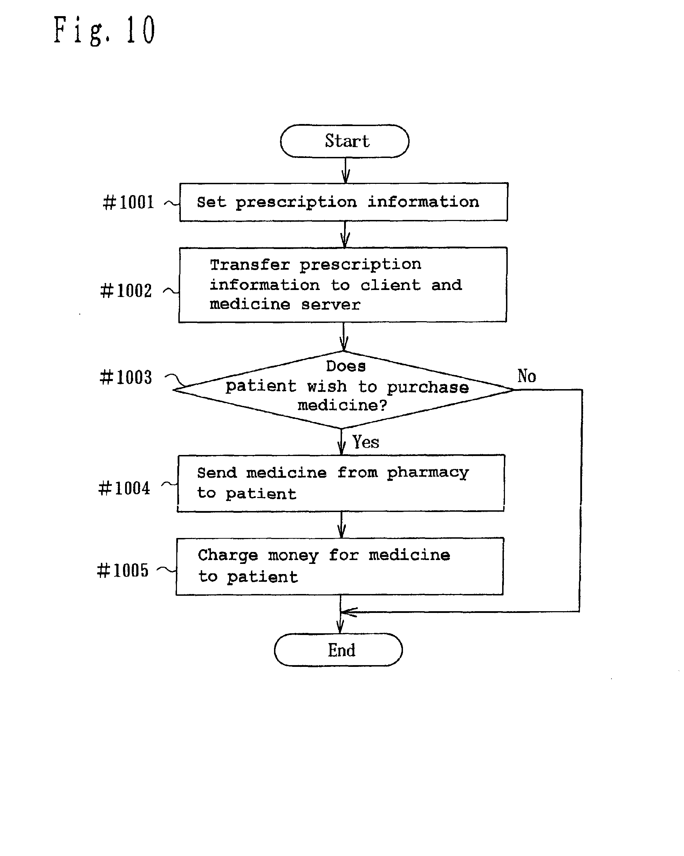

FIG. 10 is a flowchart for a medicine order reception/placement system in the medical information system shown in FIG. 11. This flowchart starts after a doctor or a nurse conducts a diagnosis of a patient.

At step #1001, as a result of the diagnosis conducted by the doctor or nurse, information regarding a necessary medicine (hereafter referred to as prescription information) is input by using the host 202 and stored in the server 203. At step #1002, this prescription information is transferred to the client 201 and the medicine server 1101.

At step #1003, the patient having received the prescription information from the client 201 makes a judgment as to whether to purchase the medicine in accordance with the direction thereof or not.

In the case when the patient decides to purchase the medicine, a request for the purchase is sent from the client 201 to the medicine server 1101, and the sequence advances to step #1004. In the case when the patient does not purchase the medicine, the processing ends.

At step #1004, the pharmacy prepares the medicine on the basis of the prescription information in the medicine server 1101 and sends the medicine to the patient by a door-to-door delivery service or the like.

At step #1005, the pharmacy charges money for the medicine to the patient.

This kind of medicine order reception/placement system saves the patient from taking trouble in getting the medicine and shortens the time for getting the medicine. Furthermore, when the patient purchases a medicine that does not require prescription information, it is possible to use a system wherein the flowchart shown in FIG. 10 begins with the step #1003, and the patient purchases the medicine online.

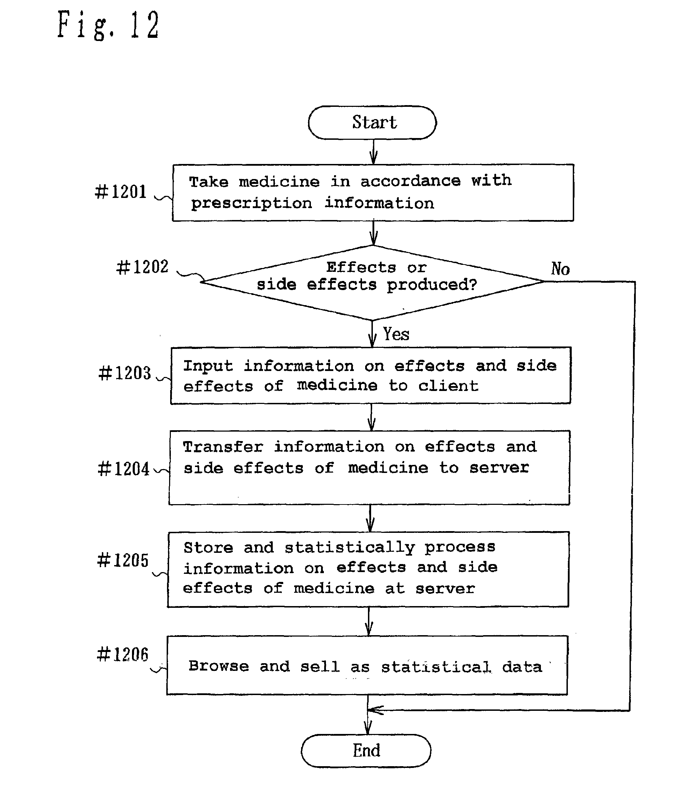

FIG. 12 is a flowchart for the registration and browsing of the information on the effects and side effects of a medicine in the medical information system shown in FIG. 11. This flowchart starts when a patient diagnosed by a doctor receives a prescription and takes a medicine. In some cases, however, this flowchart may start when a patient purchases a medicine by himself at a pharmacy without being diagnosed by a doctor.

At step #1201, the patient takes the medicine in accordance with the prescription information. At step #1202, a judgment is made as to whether the medicine taken by the patient has noteworthy effects or side effects.

If there are effects or side effects, the sequence advances to step #1203; otherwise, the processing ends.

At step #1203, the patient inputs the information on the effects and side effects of the medicine in the client 201. Furthermore, the patient inputs the medicine under consideration and the amount of the medicine having been taken. Examples of the side effects include conditions, such as headache, stomachache and nausea, and also include the degree of pain. Examples of the effects include the stoppage of bleeding, the reduction of pain and the like.

At step #1204, the input information on the effects and side effects of the medicine is transferred from the client 201 to the server 203.

Step #1205 is processed by the server 203; the transferred information on the effects and side effects of the medicine is stored, arranged and processed statistically, thereby making it possible for the user to browse the information as statistical data. By allowing the statistically processed data to be browsed, anonymity is secured, thereby avoiding the issue of the invasion of privacy.

Step #1206 is processed by the user; the user browses the above-mentioned statistical data; in the case where money is charged for the browsing, money charging is performed.

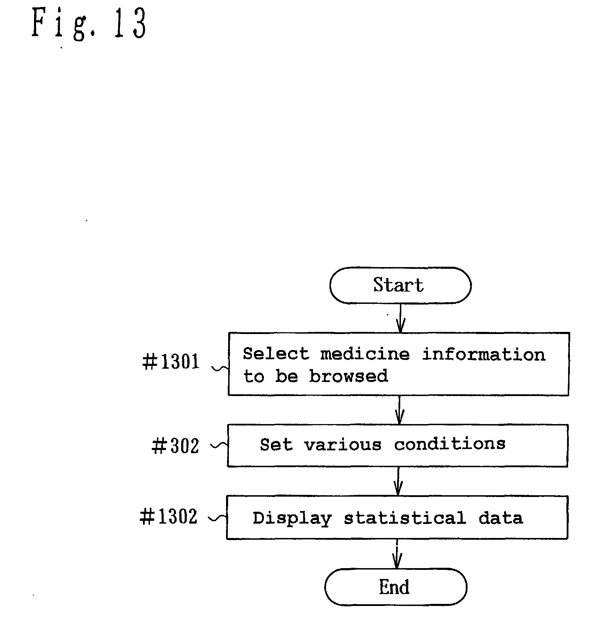

FIG. 13 is a detailed flowchart for the above-mentioned step #1206; the process thereof starts after a doctor or a nurse wishing to browse the statistical data of the information on the effects and side effects of a medicine makes a connection to the network by using the host 202 as an example of a data browsing terminal device and logs in to the server 203.

At step #1301, a medicine to be browsed is selected. At step #302, conditions for the information on the effects and side effects of the medicine to be browsed are set. The explanation regarding this condition setting is omitted since it is similar to that described above.

At step #1302, the statistical data of the information on the effects and side effects of the medicine is displayed on the basis of the input conditions.

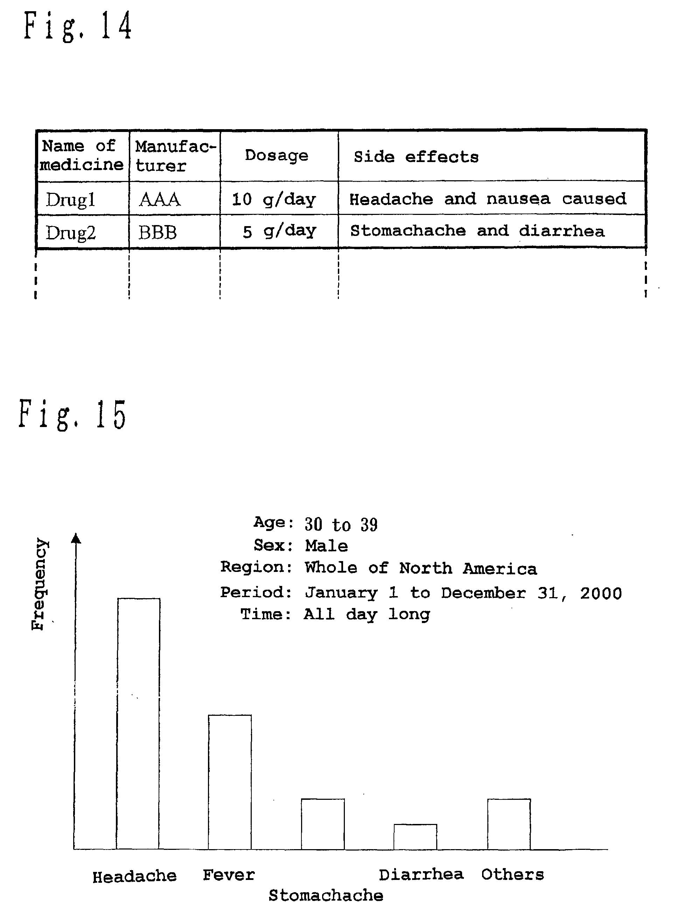

FIG. 14 shows examples of the information on the side effects of medicines, and the names of the selected medicines and the conditions due to the side effects caused by taking the medicines are described. FIG. 15 also shows examples of the information on the side effects of a medicine. The frequencies of the side effects on the basis of the input conditions are indicated visually.

As described above, the effects and side effects of the medicines taken by patients are indicated as statistical data, whereby the data can be used as a reference for the future development of medicines.

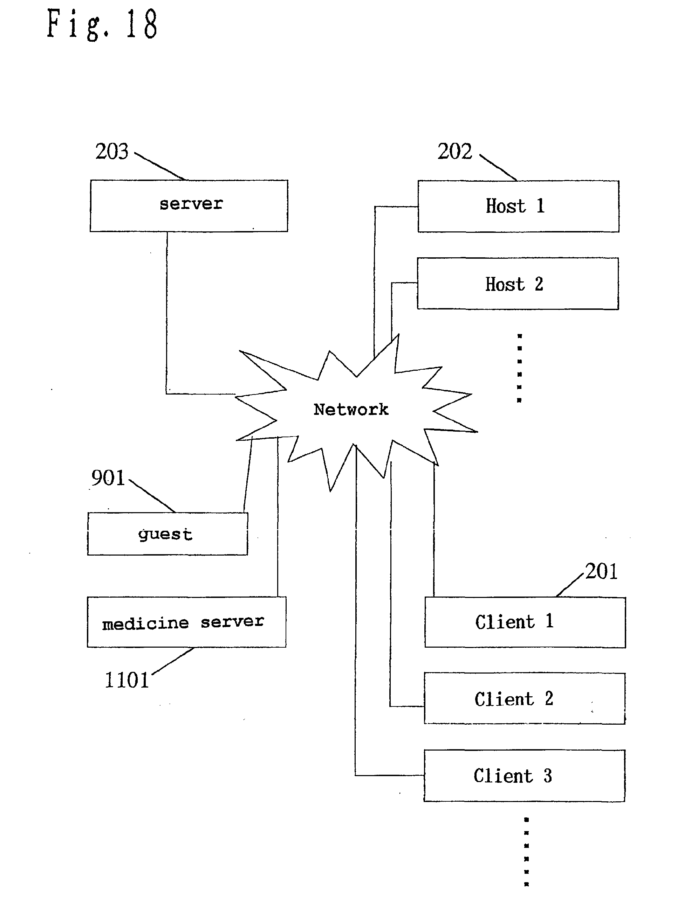

FIG. 18 is a view showing a configuration of a medical information system in accordance with a fourth preferred embodiment of the present invention. This differs from FIG. 11 in that, in addition to the hosts 202 used by doctors and nurses, a data browsing terminal device 901 (hereafter simply referred to as a guest) is connected as an example of the data browsing terminal device of the present invention.

In the above-mentioned explanations, an administrator (a doctor or a nurse) browses the statistical data by using the host 202; however, a third party (hereafter simply referred to as a user), not a patient or an administrator, that is, for example, a pharmaceutical researcher or the like, desires to browse the statistical data in some cases. In such a case, it is necessary to restrict the browsing of the statistical data or to charge money.

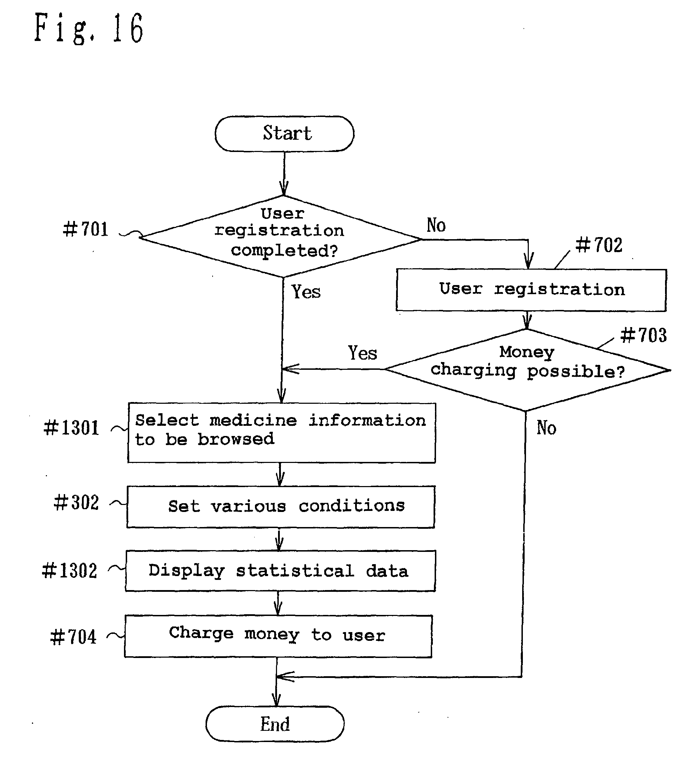

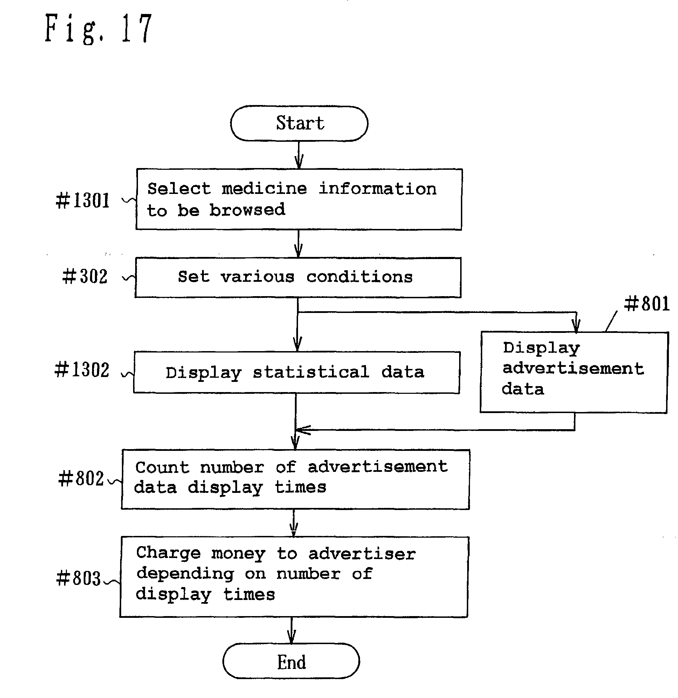

FIG. 16 is a detailed flowchart for step #1206. In the flowchart shown in FIG. 13, data browsing is available at no charge; however, the flowchart shown in FIG. 16 differs in that a charging process is added thereto. The explanation for the flowchart is omitted since it is almost similar to the explanation for the above-mentioned FIG. 7. The difference is that the vital information is handled in FIG. 7 but the information on the effects and side effects of a medicine is handled in FIG. 16. FIG. 17 is a detailed flowchart for step #104. In the flowchart of FIG. 16, money is charged to the user; however, in FIG. 17, money is charged to the advertiser. The explanation for the flowchart is omitted since it is almost similar to that for the above-mentioned FIG. 8. The difference is that the vital information is handled in FIG. 8 but the information on the effects and side effects of a medicine is handled in FIG. 17.

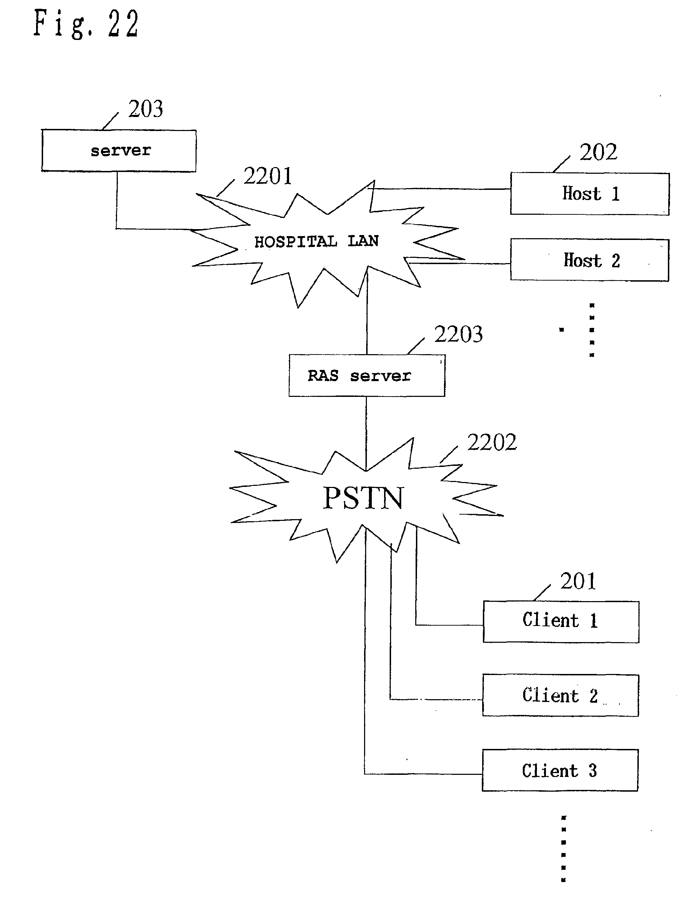

FIG. 22 is a view showing a network configuration of the medical information system in accordance with the first preferred embodiment of the present invention. In FIG. 2, only the fact that the server, hosts and clients are connected to the network is explained; however, the detailed network configuration thereof is not mentioned; in FIG. 22, a case wherein the server 203 is installed in a local area network 2201 in a hospital (hereafter simply referred to as a Hospital LAN) is described.

The client 201 is connected to a public switched telephone network 2202 (hereafter simply referred to as a PSTN), and vital information is transferred to the server. The PSTN 2202 herein mainly designates an analog telephone network; however, the network may be replaced with a digital telephone network, a cable television network, an Ethernet network, etc. In the Hospital LAN 2201, a remote access server 2203 (hereafter simply referred to as an RAS server) is installed to receive communication from the client. The RAS server 2203 performs authentication (validation of user name and password) for the connection of the client to the Hospital LAN 2201.

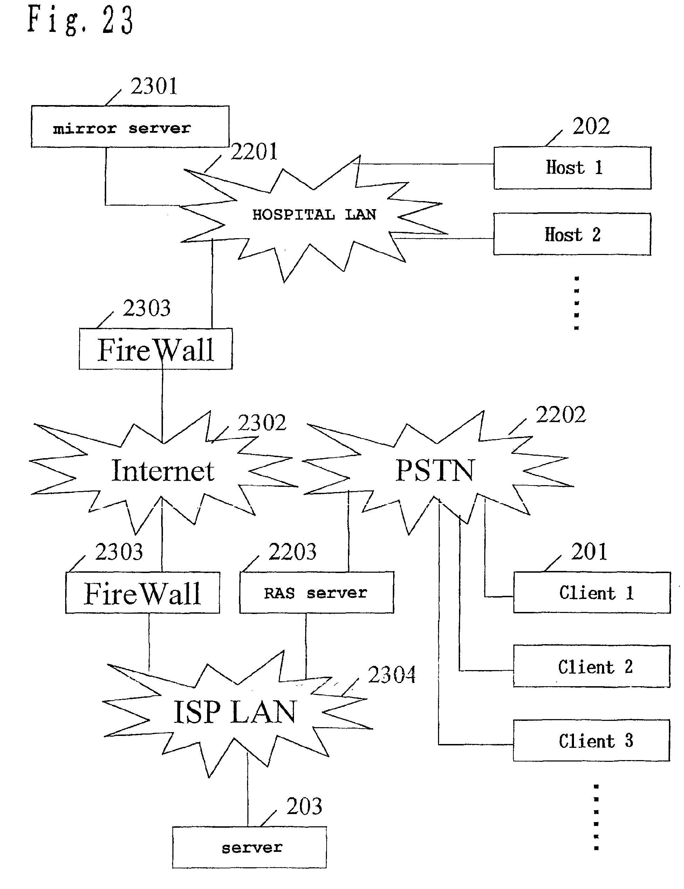

FIG. 23 is a view showing a configuration of a medical information system in accordance with a fifth preferred embodiment of the present invention. This differs from FIG. 22 in that, although the server in FIG. 22 is installed in the Hospital LAN 2201, the server in FIG. 23 is installed in an Internet service provider's local area network 2304 (hereafter simply referred to as an ISP LAN). Installing the server 203 in the ISP LAN 2304 has advantages that software and hardware can be upgraded and maintained promptly. However, a data replication terminal device 2301 (hereafter simply referred to as a mirror server) for storing the same data as that stored in the server 203 is also installed in the Hospital LAN 2201. The reason is that, when the host 202 connected to the Hospital LAN 2201 browses the vital information of the server 203, high-speed display can be made, and that, when the server 203 crashes, data recovery can be carried out smoothly. To construct the mirror server 2301, the Hospital LAN 2201 is connected to the ISP LAN 2304 through the Internet (hereafter simply referred to as the Internet), and a firewall 2303 for ensuring security (hereafter simply referred to as a FireWall) is installed at the entrance of each LAN.

The present invention relates to programs for allowing a computer to carry out the functions of all or parts of the devices or means of the medical information system and the patient terminal devices of the present invention described above, wherein the programs work in cooperation with the computer.

Furthermore, the present invention relates to a medium holding programs for allowing a computer to carry out all or parts of the functions of all or parts of the devices or means of the medical information system and the patient terminal devices of the present invention described above, wherein the medium can be read by a computer and the above-mentioned programs read by the computer carry out the above-mentioned functions in cooperation with the computer.

The parts of the devices or means of the present invention designate some devices or means in the plurality of devices or means, or designate parts of functions in one device or means.

Furthermore, a recording medium on which the programs of the present invention are recorded and which can be read by a computer is also included in the present invention.

Still further, a utilization embodiment of the programs of the present invention may be an embodiment that is recorded on a recording medium readable by a computer and operates in cooperation with the computer.

Still further, a utilization embodiment of the programs of the present invention may be an embodiment that is transmitted through a transmission medium, is read by a computer and operates in cooperation with the computer.

Still further, the recording medium includes ROM and the like, and the transmission medium includes transmission media, such as the Internet, light, radio wave and sound wave.

Still further, the computer of the present invention described above may include not only pure hardware, such as a CPU, but also firmware and OS, and may further include peripheral devices.

As described above, the configuration of the present invention may be realized by software or realized by hardware.

### Industrial Applicability

As described above, the present invention can realize a system capable of easily browsing data, such as the vital information of patients.

## Claims

1. A medical information system comprising patient terminal devices, a central control device and data browsing terminal devices, wherein
said patient terminal devices, said central control device and said data browsing terminal devices are connected on a network,
said patient terminal device has means of transferring vital information relating to the health condition of a patient to said central control device via said network and means of receiving control information from said central control device,
said central control device has means of storing said vital information transferred from said patient terminal device and arranging said information to information having anonymity, and
said data browsing terminal device has means capable of browsing all or parts of said vital information stored and arranged in said central control device.

2. A medical information system in accordance with claim 1, wherein said patient terminal device has sensors for measuring said vital information relating to the health condition of said patient.

3. A medical information system in accordance with claim 1, wherein said arranged information is statistical data.

4. Patient terminal devices mutually connected to a central control device and data browsing terminal devices on a network, comprising:
means, having a function of measuring vital information relating to the health condition of a patient, of transferring said vital information to said central control device via said network, and
means of receiving control information from said central control device, wherein
said central control device has means of storing said vital information transferred from said patient terminal device and arranging said information to information having anonymity, and
said data browsing terminal device has means capable of browsing all or parts of said vital information stored and arranged in said central control device.

5. A medical information system in accordance with claim 1, wherein, when said vital information is browsed from said data browsing terminal device, money is charged by said central control device to the person who browses.

6. A medical information system in accordance with claim 1, having means of displaying advertisement data at the same time when said vital information is browsed from said data browsing terminal device, wherein money is charged to the advertiser thereof on the basis of the number of times said advertisement data is displayed.

7. A medical information system comprising patient terminal devices, a central control device, a medicine control terminal device and administrator terminal devices, wherein said patient terminal devices, said central control device, said medicine control terminal device and said administrator terminal devices are connected on a network, wherein
said administrator terminal device has means of inputting prescription information and means of transferring said prescription information to said patient terminal devices and said medicine control terminal device via said network,
said patient terminal device has means of transferring a request for purchasing a medicine to said medicine control terminal device, and
said medicine control terminal device has means of identifying and outputting said prescription information corresponding to the patient when said request for purchasing the medicine is made.

8. Patient terminal devices connected to a central control device, a medicine control terminal device and administrator terminal devices on a network, comprising:
means of transferring a requests for purchasing a medicine to said medicine control terminal device, wherein
said administrator terminal device has means of inputting prescription information and means of transferring said prescription information to said patient terminal devices and said medicine control terminal device via said network, and
said medicine control terminal device has means of identifying and outputting said prescription information corresponding to the patient when said request for purchasing a medicine is made.

9. A medical information system comprising patient terminal devices, a central control device and data browsing terminal devices, wherein
said patient terminal devices, said central control device and said data browsing terminal devices are connected on a network,
said patient terminal device has means of allowing a patient to input the information on the effects and side effects of a medicine and means of transferring said information on the effects and side effects to said central control device via said network,
said central control device has means of storing and arranging said information on the effects and side effects, and
said data browsing terminal device has means capable of browsing all or parts of said information on the effects and side effects of the medicine stored and arranged in said central control device.

10. Patient terminal devices connected to a central control device and data browsing terminal devices on a network, comprising:
means of allowing a patient to input the information on the effects and side effects of a medicine and means of transferring said information on the effects and side effects to said central control device via said network, wherein
said central control device has means of storing and arranging said information on the effects and side effects, and
said data browsing terminal device has means capable of browsing all or parts of said information on the effects and side effects of the medicine stored and arranged in said central control device.

11. A medical information system in accordance with claim 9, wherein, when said information on the effects and side effects is browsed by said data browsing terminal device, money is charged by said central control device to the person who browses.

12. A medical information system in accordance with claim 9, having means of displaying advertisement data at the same time when said information on the effects and side effects is browsed by said data browsing terminal device, wherein money is charged to the advertiser thereof on the basis of the number of times said advertisement data is displayed.

13. A medical information system comprising patient terminal devices, a central control device, data browsing terminal devices and a data replication terminal device, wherein
said patient terminal devices, said central control device, said data browsing terminal devices and said data replication terminal device are connected on a network,
said patient terminal device has means of transferring vital information relating to the health condition of a patient to said central control device via said network and means of receiving control information from said central control device,
said central control device has means of storing said vital information transferred from said patient terminal device and arranging said information to information having anonymity,
said data browsing terminal device has means capable of browsing all or parts of said vital information stored and arranged in said central control device, and
said data replication terminal device has means capable of storing the same data as the data stored and arranged in said central control device.

14. Patient terminal devices connected to a central control device, data browsing terminal devices and a data replication terminal device on a network, comprising:
means of transferring vital information relating to the health condition of a patient to said central control device via said network, and
means of receiving control information from said central control device, wherein
said central control device has means of storing said vital information transferred from said patient terminal device and arranging said information to information having anonymity,
said data browsing terminal device has means capable of browsing all or parts of said vital information stored and arranged in said central control device, and
said data replication terminal device has means capable of storing the same data as the data stored and arranged in said central control device.

15. In a medical information system in accordance with claim 1, programs for activating a computer, as all or parts of:
means of transferring said vital information relating to the health condition of a patient to said central control device via said network and means of receiving said control information from said central control device, in said patient terminal device,
means of storing said vital information transferred from said patient terminal device and arranging said information to information having anonymity, in said central control device, and
means capable of browsing all or parts of said vital information stored and arranged in said central control device,- in said data browsing terminal device.

16. In a patient terminal device in accordance with claim 4, programs for activating a computer, as all or parts of:
means, having a function of measuring said vital information relating to the health condition of a patient, of transferring said vital information to said central control device via said network, and
means of receiving said control information from said central control device.

17. In a medical information system in accordance with claim 7, programs for activating a computer, as all or parts of:
means of inputting said prescription information and means of transferring said prescription information to said patient terminal devices and said medicine control terminal device via said network, in said administrator terminal device,
means of transferring said request for purchasing a medicine to said medicine control terminal device, in said patient terminal device, and
means of identifying and outputting said prescription information corresponding to the patient when said request for purchasing the medicine is made, in said medicine control terminal device.

18. In a patient terminal device in accordance with claim 8, programs for activating a computer, as all or parts of means of transferring said request for purchasing a medicine to said medicine control terminal device.

19. In a medical information system in accordance with claim 9, programs for activating a computer, as all or parts of:
means of allowing a patient to input said information on the effects and side effects of a medicine and means of transferring said information on effects and side effects to said central control device via said network, in said patient terminal device,
means of storing and arranging said information on the effects and side effects, in said central control device, and
means capable of browsing all or parts of said information on the effects and side effects of the medicine stored and arranged in said central control device, in said data browsing terminal device.

20. In a patient terminal device in accordance with claim 10, programs for activating a computer, as all or parts of means of allowing a patient to input said information on the effects and side effects of a medicine and means of transferring said information on the effects and side effects to said central control device via said network.

21. In a medical information system in accordance with claim 13, programs for activating a computer, as all or parts of:
means of transferring said vital information relating to the health condition of a patient to said central control device via said network and means of receiving said control information from said central control device, in said patient terminal device,
means of storing said vital information transferred from said patient terminal device and arranging said information to information having anonymity, in said central control device,
means capable of browsing all or parts of said vital information stored and arranged in said central control device, in said data browsing terminal device, and
means capable of storing the same data as the data stored and arranged in said central control device, in said data replication terminal device.

22. In a patient terminal device in accordance with claim 14, programs for activating a computer, as all or parts of:
means of transferring said vital information relating to the health condition of a patient to said central control device via said network, and
means of receiving said control information from said central control device.

23. In a medical information system in accordance with claim 1, a computer processable medium that holds programs for activating a computer, as all or parts of:
means of transferring said vital information relating to the health condition of a patient to said central control device via said network and means of receiving said control information from said central control device, in said patient terminal device,
means of storing said vital information transferred from said patient terminal device and arranging said information to information having anonymity, in said central control device, and
means capable of browsing all or parts of said vital information stored and arranged in said central control device, in said data browsing terminal device.

24. In a patient terminal device in accordance with claim 4, a computer processable medium that holds programs for activating a computer, as all or parts of:
means, having a function of measuring said vital information relating to the health'condition of a patient, of transferring said vital information to said central control device via said network, and
means of receiving said control information from said central control device.

25. In a medical information system in accordance with claim 7, a computer processable medium that holds programs for activating a computer, as all or parts of:
means of inputting said prescription information and means of transferring said prescription information to said patient terminal devices and said medicine control terminal device via said network, in said administrator terminal device,
means of transferring said request for purchasing a medicine to said medicine control terminal device, in said patient terminal device, and
means of identifying and outputting said prescription information corresponding to the patient when said request for purchasing a medicine is made, in said medicine control terminal device.

26. In a patient terminal device in accordance with claim 8, a computer processable medium that holds programs for activating a computer, as all or parts of means of transferring said request for purchasing a medicine to said medicine control terminal device.

27. In a medical information system in accordance with claim 9, a computer processable medium that holds programs for activating a computer, as all or parts of:
means of allowing a patient to input said information on the effects and side effects of a medicine and means of transferring said information on the effects and side effects to said central control device via said network, in said patient terminal device, in said patient terminal device,
means of storing and arranging said information on the effects and side effects, in said central control device, and
means capable of browsing all or parts of said information on the effects and side effects of the medicine stored and arranged in said central control device, in said data browsing terminal device.

28. In a patient terminal device in accordance with claim 10, a computer processable medium that holds programs for activating a computer, as all or parts of means of allowing a patient to input said information on the effects and side effects of a medicine and means of transferring said information on the effects and side effects to said central control device via said network.

29. In a medical information system in accordance with claim 13, a computer processable medium that holds programs for activating a computer, as all or parts of:
means of transferring said vital information relating to the health condition of a patient to said central control device via said network and means of receiving said control information from said central control device, in said patient terminal device,
means of storing said vital information transferred from said patient terminal device and arranging said information to information having anonymity, in said central control device,
means capable of browsing all or parts of said vital information stored and arranged in said central control device, in said data browsing terminal device, and
means capable of storing the same data as the data stored and arranged in said central control device, in said data replication terminal device.

30. In a patient terminal device in accordance with claim 14, a computer processable medium that holds programs for activating a computer, as all or parts of:
means, having a function of measuring said vital information relating to the health condition of a patient, of transferring said vital information to said central control device via said network, and
means of receiving said control information from said central control device.
